# EUROPÄISCHE PATENTSCHRIFT

(11) **EP 3 378 401 B1**
(45) Veröffentlichungstag und Bekanntmachung des Hinweises auf die Patenterteilung: **12.02.2025**
(21) Anmeldenummer: 17162573.4
(22) Anmeldetag: 23.03.2017
(51) Int. Cl.: A61B 6/00, A61B 6/02, A61B 6/12, G06T 11/00, A61B 6/50

(54) **DARSTELLUNG EINES INTERESSIERENDEN BEREICHS**
REPRESENTATION OF AN AREA OF INTEREST
PRÉSENTATION D'UNE ZONE D'INTÉRÊT

(43) Veröffentlichungstag der Anmeldung: 26.09.2018
(73) Patentinhaber: Siemens Healthineers AG, 91301 Forchheim (DE)
(72) Erfinder: Jerebko, Anna, 91353 Hausen (DE); Veitenhansl, Stefan, 90427 Nürnberg (DE)
(74) Vertreter: Siemens Healthineers Patent Attorneys

(56) Entgegenhaltungen:
- DE-B4- 102011 087 337
- US-A1- 2016 089 090
- US-A1- 2016 235 380

## Beschreibung

Die Erfindung betrifft ein Verfahren zur Darstellung eines interessierenden Bereichs, eine Bildrekonstruktionseinrichtung und ein Tomosynthesesystem.

Zur Früherkennung von Mammakarzinomen gibt es eine Vielzahl an Diagnosemethoden. Neben der klassischen ärztlichen Untersuchung durch Abtasten werden häufig zweidimensionale Röntgenaufnahmen als Projektionen der Brust, sogenannte Mammogramme, angefertigt. Problematisch ist dabei, dass durch die Überlagerung verschiedener Gewebestrukturen krankhafte Veränderungen im Gewebe häufig verdeckt und daher nicht erkannt werden. Dieses Problem versucht man zu kompensieren, indem man die Brust aus zwei unterschiedlichen Winkeln, beispielsweise einmal craniocaudal (CC), d. h. in Richtung vom Kopf zu den Füßen, und einmal im 45°-Winkel dazu (mediolateral oblique, MLO) aufnimmt.

Die Projektionsbilder geben allerdings noch keinen Aufschluss über die Dignität der Veränderung. Es bleibt also unklar, ob es sich um eine gutartige oder bösartige Gewebeveränderung handelt. Um dies aufzuklären, muss in der Regel im Rahmen einer Biopsie eine Gewebeprobe entnommen werden, deren Änderungen im feingeweblichen Aufbau folgend histologisch untersucht werden. Für die Probeentnahme ist es möglich, die Gewebeveränderung anhand der zweier stereotaktischer Aufnahmen zu lokalisieren, die bei komprimierter Brust und stationärem Detektor unter Akquisitionswinkeln von +15° und -15° der Röntgenquelle zu einer Normalen der Detektionsfläche aufgenommen werden.

Des Weiteren ist die Technik der sogenannten Tomosynthese bekannt. Sie beschreibt ein Bildgebungsverfahren, bei dem die Brust aus einer Vielzahl von unterschiedlichen Winkeln aufgenommen wird. Im Rahmen eines Tomosynthesescans werden dazu bei komprimierter Brust beispielsweise Projektionen in Winkeln von 15 bis 50 Grad der Röntgenquelle zu einer Normalen der stationären Detektionsfläche erfasst, wobei die Gesamtdosis in etwa der zweier klassischer zweidimensionalen Mammogramme (also z. B. CC und MLO) entspricht. Aus den erfassten Projektionsdaten werden üblicherweise Bilder für einzelne Schichten des Brustgewebes errechnet, d. h. rekonstruiert. Um einen Volumendatensatz eines zu untersuchenden Bereichs aus den erfassten Projektionsdaten zu rekonstruieren, kommt häufig das Verfahren der gefilterten Rückprojektion zum Einsatz. Der resultierende Volumendatensatz kann zu Diagnosezwecken schichtweise betrachtet werden. Da Schichten über und unter der jeweils zur Ansicht ausgewählten Schicht bei der Befundung ausgeblendet werden können, sind krankhafte Gewebeveränderungen in der Regel leichter zu erkennen. Allerdings werden dabei die Schichten aus lediglich einer Richtung dargestellt, sodass es sich je nach Richtung der Gewebeveränderung weiterhin als schwierig erweisen kann, diese festzustellen und exakt zu lokalisieren.

Während einer anschließenden Biopsie mittels einer Biopsienadel werden üblicherweise vor bzw. nach einer tatsächlichen Probeentnahme Aufnahmen zur Kontrolle der Position der Nadel erstellt. Aufgrund der in den Strahlengang eingebrachten Nadel gestaltet sich dabei die Aufnahme eines vollständigen Tomosynthesescans schwierig, da insbesondere durch große Vakuumbiopsiesysteme störende Artefakte verursacht werden. Häufig werden daher im rein stereotaktischen Betrieb als Kontrollaufnahmen lediglich zwei Projektionen aus festgelegten Winkeln angefertigt. Daraus lässt sich allerdings nur schwer ein guter räumlicher Eindruck von der Position der Nadel gewinnen.

Aus der Druckschrift US 2016/235380 A1 ist ein Verfahren zur Durchführung eines Eingriffs an der Brust eines Patienten bekannt, welcher das Zusammendrücken der Brust des Patienten mit einem Paddel eines Abbildungssystems und die Durchführung eines anfänglichen Abbildungsvorgangs an der Brust des Patienten umfasst. Die anfängliche Bildgebungsprozedur umfasst die Bildgebung der Brust mit einer Röntgenquelle bei einer ersten Energie, um ein erstes Bild zu erhalten, und einer zweiten Energie, um ein zweites Bild zu erhalten. Aus dem ersten Bild und dem zweiten Bild wird ein zusammengesetztes Bild erzeugt und angezeigt. Ein interessanter Bereich auf dem zusammengesetzten Bild wird anvisiert und eine Biopsie wird durchgeführt.

Aus der Druckschrift US 2016/089090 A1 ist ein Strahlungsabbildungssystem, eine Bildverarbeitungsvorrichtung, ein Strahlungsabbildungsverfahren und ein nicht-transitorisches computerlesbares Aufzeichnungsmedium mit einem darauf aufgezeichneten Bildverarbeitungsprogramm bekannt, die die Bestätigung einer Positionsbeziehung zwischen einem Objekt von Interesse und einer Biopsienadel erleichtern. In dem System, das eine Strahlungsbildgebungsvorrichtung als eine Mammographievorrichtung verwendet, wird bei der Durchführung einer Biopsie einer Brust einer Person die Positionsbeziehung unter Verwendung eines Strahlungsbildes (Projektionsbildes) bestätigt, das durch Tomosynthese-Bildgebung erhalten wird. Eine Steuereinheit rekonstruiert das Projektionsbild, um ein tomographisches Bild in einem Zustand zu erzeugen, in dem die Nadel in die Brust eingeführt ist, und erzeugt ein Reprojektionsbild in einem vorgegebenen Winkel aus dem tomographischen Bild. Die Steuereinheit extrahiert ein Bild der Nadel aus dem Projektionsbild, fügt das extrahierte Bild der Nadel in das Projektionsbild ein, während es ausgerichtet wird, und zeigt das Projektionsbild an.

Es ist daher eine Aufgabe der vorliegenden Erfindung, trotz einem in das Untersuchungsobjekt eingebrachten Untersuchungsinstrument einen verbesserten räumlichen Eindruck von einem interessierenden Bereich im Inneren des Untersuchungsobjekts mittels eines Tomosynthesesystems zu ermöglichen.

Diese Aufgabe wird durch ein Verfahren zur Darstellung gemäß Patentanspruch 1, eine Bildrekonstruktionseinrichtung gemäß Patentanspruch 7 und ein Tomosynthesesystem gemäß Patentanspruch 8 gelöst.

Das eingangs genannte Verfahren zur Darstellung eines interessierenden Bereichs innerhalb eines Untersuchungsobjekts, in das ein Untersuchungsinstrument eingebracht ist, wird mittels eines Tomosynthesesystems durchgeführt. Es umfasst zumindest die Schritte gemäss Anspruch 1.

Bei dem Untersuchungsobjekt kann es sich beispielsweise um ein Organ einer menschlichen oder tierischen Patientin handeln, bevorzugt ist das Untersuchungsobjekt jedoch eine Brust einer menschlichen Patientin. Im Folgenden werden daher ohne Beschränkung der Allgemeinheit die Begriffe "Untersuchungsobjekt" und "Brust der Patientin" synonym verwendet. Bei dem interessierenden Bereich handelt es sich beispielsweise um eine Mikrokalzifikation, die sich im Inneren des Untersuchungsobjekts, also innerhalb der Brust befindet. Mikrokalzifikationen deuten potenziell auf ein Karzinom hin, da proliferierende Zellen mit der Zeit nekrotisch werden und Kalk anlagern. Dieser Bereich wird mit Hilfe des erfindungsgemäßen Verfahrens derart dargestellt bzw. visualisiert, dass eine gute räumliche Positionsbestimmung bzw. Lokalisierung ermöglicht wird. Dies ist insbesondere bei einer Biopsie von Vorteil, da die Positionierung des Untersuchungsinstruments, also z. B. der Biopsienadel, erfindungsgemäß räumlich gut bestimmt und dadurch visuell überprüft werden kann, wie später noch näher erläutert wird.

Die dazu erforderlichen Rohdaten umfassen bevorzugt eine Mehrzahl von Projektionsaufnahmen, also - anders als bei einer stereotaktischen Untersuchung - bevorzugt mehr als zwei.

Sie können grundsätzlich erfasst werden, indem sie z. B. aus einer Speichereinrichtung oder über ein Netzwerk abgerufen werden, bevorzugt werden sie jedoch im Rahmen eines vorbereitenden Schrittes mittels eines Tomosynthesegeräts akquiriert. Dazu wird die Mehrzahl von Projektionsaufnahmen des Untersuchungsobjekts erstellt, indem das Untersuchungsobjekt von Röntgenstrahlung durchdrungen wird. Das Untersuchungsobjekt ist dafür zwischen einer Röntgenquelle und einem nicht rotierenden Detektor angeordnet. Die Röntgenquelle emittiert Röntgenstrahlung, die der Detektor nach einer Projektion durch das Untersuchungsobjekt erfasst. Die Röntgenquelle ist dabei bevorzugt in einem bestimmten Winkelbereich gegenüber einer sich senkrecht zum Detektor erstreckenden Mittelachse verstellbar. Die Mittelachse kann sich dabei in einer beliebigen Richtung in Relation zum Körper der Patientin erstrecken. Bevorzugt entspricht sie einer craniocaudalen Richtung (CC) oder einer mediolateral oblique Richtung (MLO). Die Röntgenquelle projiziert die Röntgenstrahlung somit aus unterschiedlichen definierten Akquisitionswinkeln durch das Untersuchungsobjekt auf den Detektor, der die Projektionsaufnahmen der Brust als Rohdatensätze erfasst. Alternativ können aber sowohl der Detektor als auch die Röntgenquelle um das Untersuchungsobjekt rotiert werden und auf diese Weise Projektionsaufnahmen als Rohdatensätze aus unterschiedlichen Akquisitionswinkeln aufnehmen.

Die Rohdaten werden erfindungsgemäß - bevorzugt nur - aus seitlichen Akquisitionswinkeln aufgenommen, die Projektion erfolgt also schräg zur Mittelachse. Im Gegensatz zu einem üblichen Tomosynthesescan werden also erfindungsgemäß die Akquisitionswinkel in einem Bereich um die Mittelachse ausgelassen und es werden keine mittleren Projektionsaufnahmen erzeugt. Denn im Bereich der Mittelachse ist erfindungsgemäß das Untersuchungsinstrument, also z. B. eine Biopsieeinrichtung, insbesondere mit einer Vakuumbiopsienadel angeordnet. Bei den mittleren Projektionsaufnahmen würden dadurch Artefakte bei der Rekonstruktion verursacht, was die Bildgebung bzw. Rekonstruktion erschwert. Durch die erfindungsgemäße

Nutzung der seitlichen Akquisitionswinkel bei der Erfassung der Rohdaten können diese

Störeinflüsse weitgehend vermieden werden. Zugleich wird dadurch die Strahlenbelastung für den Patienten vorteilhafterweise verringert.

Die einzelnen Projektionsaufnahmen werden dabei bevorzugt mit einer so geringen Strahlendosis akquiriert, dass für die gesamte Akquisition vorteilhafterweise in etwa nur die Strahlendosis eines üblichen Mammogramms verwendet wird. Dadurch ist auf den einzelnen Projektionsaufnahmen der Kontrast nicht ausreichend, dass Details des Untersuchungsobjekts, insbesondere der interessierende Bereich, zu erkennen sind. Dafür werden erfindungsgemäß aus den Rohdaten zunächst synthetische Projektionen ermittelt.

Aus den Rohdaten können die synthetischen Projektionen beispielsweise erzeugt werden, indem zunächst ein Volumendatensatz mittels bekannter Verfahren wie z. B. der gefilterten Rückprojektion rekonstruiert wird. Der Volumendatensatz wird folgend im Rahmen einer Vorwärtsprojektion auf einen virtuellen Detektor projiziert, wodurch eine virtuelle bzw. synthetische Projektion erhalten wird. Bevorzugt wird jedoch keine vollständige Rekonstruktion eines Volumendatensatzes vorgenommen, sondern es werden lediglich einzelne, besonders bevorzugt genau eine, dünne Schicht(en) rekonstruiert, aus denen mittels Vorwärtsprojektionen die virtuellen Projektionen gewonnen werden. Dies ist beispielsweise in der Schrift US 2014/0294138 A1 beschrieben. Alternativ werden die synthetischen Projektionen werden direkt aus den Rohdaten rekonstruiert, wodurch gegenüber einer vollständigen Volumenrekonstruktion vorteilhafterweise Rechenleistung eingespart werden kann. Dies ist beispielsweise in der Schrift DE 10 2011 087 337 B4 beschrieben.

Dabei kann die Anzahl der synthetischen Projektionen gleich der Anzahl der als Rohdaten akquirierten Projektionsaufnahmen sein, sie kann aber auch je nach Bedarf und Art der Rekonstruktion verschieden sein, also mehr oder weniger Projektionsbilder umfassen.

Die Ausgabe der synthetischen Projektionen kann beispielsweise in einen Speicher einer Recheneinheit (Computer), über ein Netzwerk und/oder auf einem Bildschirm erfolgen. Die synthetischen Projektionen werden dabei bevorzugt als begrenzt rotierendes Mammogramm dargestellt, das besonders bevorzugt im Wesentlichen nur im gleichen Winkelbereich wie die Akquisitionswinkel rotiert. Dadurch wird im Vergleich zu einer Schichtbilddarstellung eines üblichen Tomosynthesescans eine bessere räumliche Zuordnung sowie höhere Genauigkeit der Lokalisierung in z-Richtung, also senkrecht zu den Bildschichten, ermöglicht.

Die eingangs genannte Bildrekonstruktionseinrichtung zur Darstellung eines interessierenden Bereichs innerhalb eines Untersuchungsobjekts, in das ein Untersuchungsinstrument eingebracht ist, umfasst eine Erfassungseinheit, eine Ermittlungseinheit und eine Ausgabeeinheit. Sie ist so ausgebildet, dass sie die Schritte eines erfindungsgemäßen Verfahrens zur Darstellung ausführt.

Das eingangs genannte Tomosynthesesystem umfasst ein Tomosynthesegerät mit einer Quelle-Detektor-Anordnung, die zur Aufnahme von Rohdaten des Untersuchungsobjekts aus definierten Akquisitionswinkeln ausgebildet ist, sowie eine erfindungsgemäße Bildrekonstruktionseinrichtung.

Bevorzugt umfasst das Tomosynthesesystem auch eine Biopsieeinrichtung mit einem Untersuchungsinstrument, d. h. mit einer Biopsienadel, die im Rahmen einer nicht von der Erfindung umfassten Biopsie zur Entnahme einer Gewebeprobe in das Untersuchungsobjekt eingebracht wird. Während der Biopsie kann dann mit dem Tomosynthesesystem das erfindungsgemäße Verfahren durchgeführt werden, sodass zur Kontrolle und/oder zur Dokumentation der Untersuchung eine möglichst umfangreiche, detaillierte und intuitive Visualisierung erzielt wird, um eine erfolgreiche Untersuchung zu erleichtern.

Nach Maßgabe der aus dem erfindungsgemäßen Verfahren gezogenen Informationen kann die Biopsieeinrichtung zum Beispiel vor der eigentlichen - nicht von der Erfindung umfassten - Probenentnahme automatisch oder durch Bedienpersonal nachjustiert werden, was vorteilhafterweise eine genauere Positionierung beim Eingriff gewährleistet.

Die wesentlichen Komponenten der erfindungsgemäßen Bildrekonstruktionseinrichtung können zum überwiegenden Teil in Form von Softwarekomponenten ausgebildet sein. Grundsätzlich können diese Komponenten aber auch zum Teil, insbesondere wenn es um besonders schnelle Berechnungen geht, in Form von softwareunterstützter Hardware, beispielsweise FPGAs oder dergleichen, realisiert sein. Ebenso können die benötigten Schnittstellen, beispielsweise wenn es nur um eine Übernahme von Daten aus anderen Softwarekomponenten geht, als Softwareschnittstellen ausgebildet sein. Sie können aber auch als hardwaremäßig aufgebaute Schnittstellen ausgebildet sein, die durch geeignete Software angesteuert werden.

Insbesondere kann die erfindungsgemäße Bildrekonstruktionseinrichtung Teil eines Benutzerterminals eines Tomosynthesesystems sein.

Eine weitgehend softwaremäßige Realisierung hat den Vorteil, dass auch schon bisher verwendete Bildrekonstruktionseinrichtungen auf einfache Weise durch ein Software-Update nachgerüstet werden können, um auf die erfindungsgemäße Weise zu arbeiten. Insofern wird die Aufgabe auch durch ein entsprechendes Computerprogrammprodukt mit einem Computerprogramm gelöst, welches direkt in eine Speichereinrichtung einer Bildrekonstruktionseinrichtungen eines Tomosynthesesystems ladbar ist, mit Programmabschnitten, um alle Schritte des erfindungsgemäßen Verfahrens auszuführen, wenn das Programm in der Bildrekonstruktionseinrichtung ausgeführt wird. Ein solches Computerprogrammprodukt kann neben dem Computerprogramm gegebenenfalls zusätzliche Bestandteile wie z. B. eine Dokumentation und/oder zusätzliche Komponenten, auch Hardware-Komponenten, wie z.B. Hardware-Schlüssel (Dongles etc.) zur Nutzung der Software, umfassen.

Zum Transport zur Bildrekonstruktionseinrichtung und/oder zur Speicherung an oder in der Bildrekonstruktionseinrichtung kann ein computerlesbares Medium, beispielsweise ein Memorystick, eine Festplatte oder ein sonstiger transportabler oder fest eingebauter Datenträger dienen, auf welchem die von einer Rechnereinheit der Bildrekonstruktionseinrichtung einlesbaren und ausführbaren Programmabschnitte des Computerprogramms gespeichert sind. Die Rechnereinheit kann z.B. hierzu einen oder mehrere zusammenarbeitende Mikroprozessoren oder dergleichen aufweisen.

Weitere, besonders vorteilhafte Ausgestaltungen und Weiterbildungen der Erfindung ergeben sich aus den abhängigen Ansprüchen sowie der nachfolgenden Beschreibung, wobei die unabhängigen Ansprüche einer Anspruchskategorie auch analog zu den abhängigen Ansprüchen oder Beschreibungsteilen einer anderen Anspruchskategorie weitergebildet sein können und insbesondere auch einzelne Merkmale verschiedener Ausführungsbeispiele bzw. Varianten zu neuen Ausführungsbeispielen bzw. Varianten kombiniert werden können.

Da aufgrund der Anordnung des Untersuchungsinstruments insbesondere die mittleren Projektionsaufnahmen bei der Akquisition der Rohdaten zum Entstehen von Artefakten bei der Bildgebung beitragen, werden die Rohdatensätze bevorzugt mit Akquisitionswinkeln von mindestens 10°, besonders bevorzugt mindestens 15°, zu einer Mittelachse erfasst. Die Mittelachse erstreckt sich dabei, wie zuvor bereits erwähnt, in einer Körperrichtung der Patientin z. B. craniocaudal (CC) oder mediolateral oblique (MLO).

Die Rohdatensätze werden vorzugsweise mit Akquisitionswinkeln von höchstens 60°, besonders bevorzugt höchstens 45°, ganz besonders bevorzugt höchstens 25°, zu einer Mittelachse erfasst. Mit den aus diesen Rohdaten ermittelbaren synthetischen Projektionen lässt sich bereits ein guter räumlicher Eindruck des interessierenden Bereichs gewinnen.

Bevorzugt beträgt die Anzahl der seitlichen Akquisitionswinkel bzw. die Anzahl der als Rohdaten akquirierten Projektionsaufnahmen mindestens 10, besonders bevorzugt 20. Bei 10 Projektionsaufnahmen werden beispielsweise je 5 Aufnahmen pro Seite in Bezug zur Mittelachse in einem Winkelbereich von 15° bis 25° zur Mittelachse mit Winkelabständen von 2° akquiriert. Bei 20 Projektionsaufnahmen werden beispielsweise je 10 Aufnahmen pro Seite in Bezug zur Mittelachse in einem Winkelbereich von 15° bis 25° zur Mittelachse mit Winkelabständen von 1° akquiriert. Mit dieser Anzahl von Projektionsaufnahmen lassen sich bereits synthetische Projektionen in ausreichender Qualität erstellen und die Strahlenbelastung für die Patientin wird so vorteilhafterweise gering gehalten.

Beim erfindungsgemäßen Verfahren werden vorzugsweise zwei Rohdatensätze jeweils aus Akquisitionswinkeln erfasst, die einander - bezogen auf eine Mittelachse - gegenüberliegend angeordnet sind. Die Rohdatensätze werden also bevorzugt mit gegenüberliegenden Bereichen der Akquisitionswinkel beidseitig der Mittelachse aufgenommen bzw. akquiriert. Aus den Rohdatensätzen werden vorzugsweise zwei Bildsequenzen ermittelt, die den interessierenden Bereich von zwei unterschiedlichen Seiten bzw. aus zwei unterschiedlichen (zentralen) Richtungen rotierend darstellen. Dadurch wird eine noch bessere räumliche Lokalisierung des interessierenden Bereichs ermöglicht.

Die Ausgabe der synthetischen Projektionen umfasst bevorzugt eine Maximumintensitätsprojektion (MIP, "maximum intensity projection"). Mittels der Maximumintensitätsprojektionen können ein Volumen repräsentierende Daten räumlich besonders intuitiv dargestellt werden. Dazu werden aus den Rohdaten, die das dreidimensionale Volumen des Untersuchungsobjekts abbilden, zweidimensionale synthetische Projektionen ermittelt, indem bei der Rekonstruktion der synthetischen Projektionen entlang einer Blickrichtung (Projektionsrichtung der synthetischen Projektion) jeweils der Datenpunkt bzw. das Pixel mit der maximalen Intensität ausgewählt wird.

Zusätzlich zu der bereits beschriebenen rotierenden Darstellung kann es die räumliche Auffassung eines Bedieners noch weiter verbessen, wenn auch ein Schichtdatensatz des Untersuchungsobjekts bereitgestellt wird. Bevorzugt wurde daher bereits ein Tomosynthesescan akquiriert, bevor das Untersuchungsinstrument in das Untersuchungsobjekt eingebracht wird. Dadurch wird das Untersuchungsinstrument als Ursache von Artefakten bei der Akquisition des Tomosynthesescans vorteilhafterweise vermieden. Der Tomosynthesescan wird bevorzugt zusätzlich zu der Bildsequenz ausgegeben, sodass beide Datensätze zur Lokalisierung des interessierenden Bereichs verglichen werden können.

Die Rohdaten werden vorzugsweise vor oder nach einer Betätigung des Untersuchungsinstruments erfasst. Die Erfassung der Rohdaten kann also je nach Bedarf vor oder auch nach der Betätigung des Untersuchungsinstruments erfolgen. Bei der Betätigung des Untersuchungsinstruments handelt es sich bevorzugt um den Vorgang der Probenentnahme bei der Biopsie mit Hilfe einer Biopsienadel, den sog. "Fire". Die vor der Betätigung akquirierten Rohdaten werden dementsprechend als "Pre-Fire"-Daten bezeichnet, während es sich bei den nach der Betätigung akquirierten Rohdaten um sog. "Post-Fire"-Daten handelt. Da die Biopsienadel sowohl vor als auch nach dem Fire bereits in die Brust der Patientin eingebracht ist, würde sie bei einer herkömmlichen Bildgebung Artefakte verursachen, die die Darstellung erschweren oder ggf. unmöglich machen. Im Gegensatz dazu kann mit dem erfindungsgemäßen Verfahren die Position der Biopsienadel in Relation zum interessierenden Bereich, also z. B. der Mikrokalzifikation, auch unter diesen Bedingungen räumlich gut dargestellt werden, sodass eine verbesserte Positionskontrolle der Biopsienadel bzw. eine bessere Dokumentation des Eingriffs ermöglicht wird.

Die Erfindung wird im Folgenden unter Hinweis auf die beigefügten Figuren anhand von Ausführungsbeispielen noch einmal näher erläutert. Dabei sind in den verschiedenen Figuren gleiche Komponenten mit identischen Bezugsziffern versehen. Die Figuren sind in der Regel nicht maßstäblich. Es zeigen:
FIG 1 eine grob schematische Darstellung eines Ausführungsbeispiels eines erfindungsgemäßen Tomosynthesesystems,
FIG 2 ein schematisches Blockschaltbild eines Ausführungsbeispiels einer erfindungsgemäßen Bildrekonstruktionseinrichtung,
FIG 3 eine schematische Darstellung der Anordnung der Bereiche der Akquisitionswinkel bei einem Ausführungsbeispiel eines erfindungsgemäßen Verfahrens zur Darstellung eines interessierenden Bereichs,
FIG 4 ein Blockdiagramm eines Ausführungsbeispiels eines erfindungsgemäßen Verfahrens zur Darstellung eines interessierenden Bereichs und
FIG 5 ein Blockdiagramm eines weiteren Ausführungsbeispiels eines erfindungsgemäßen Verfahrens zur Darstellung eines interessierenden Bereichs.

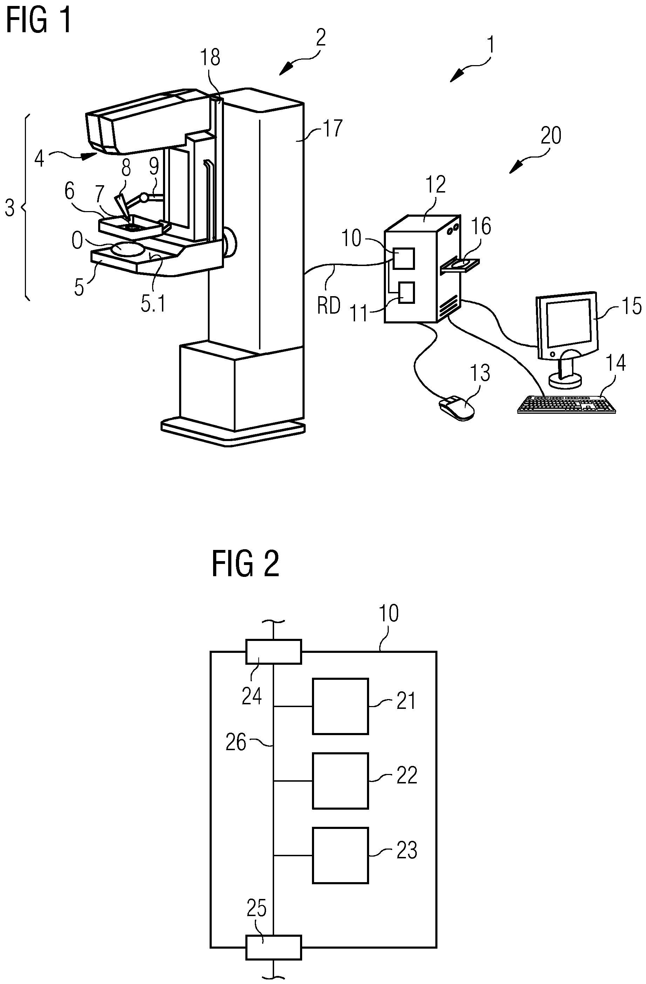

In FIG 1 ist beispielhaft und grob schematisch ein erfindungsgemäßes Tomosynthesesystem 1 gezeigt. Relative Richtungsangaben wie "oben" und "unten" etc. beziehen sich auf ein bestimmungsgemäß für den Betrieb aufgestelltes Tomosynthesesystem 1. Das Tomosynthesesystem 1 umfasst ein Tomosynthesegerät 2 und ein Rechnersystem 20. Das Tomosynthesegerät 2 weist eine Standsäule 17 und Quelle-Detektor-Anordnung 3 auf, die wiederum eine Röntgenstrahlungsquelle 4 (folgend auch Röntgenquelle 4) und einen Detektor 5 mit einer Detektionsfläche 5.1 umfasst. Die Standsäule 17 steht im Betrieb auf dem Untergrund. Mit ihr ist die Quelle-Detektor-Anordnung 3 verschiebbar verbunden, sodass die Höhe der Detektorfläche 5.1, also der Abstand zum Untergrund, auf eine Brusthöhe einer Patientin eingestellt werden kann.

Eine Brust O der Patientin (hier schematisch dargestellt) liegt als Untersuchungsobjekt O für eine Untersuchung oberseitig auf der Detektorfläche 5.1 auf. Über der Brust O und der Detektorfläche 5.1 ist eine Platte 6 angeordnet, die verschiebbar mit der Quelle-Detektor-Anordnung 3 verbunden ist. Für die Untersuchung wird die Brust O komprimiert und zugleich fixiert, indem die Platte 6 auf sie herabgesenkt wird, sodass auf die Brust O zwischen Platte 6 und Detektorfläche 5.1 ein Druck ausgeübt wird. Die Platte 6 weist mittig eine kreisförmige Ausnehmung 7 auf, durch die die Brust O für die Untersuchung zugänglich wird.

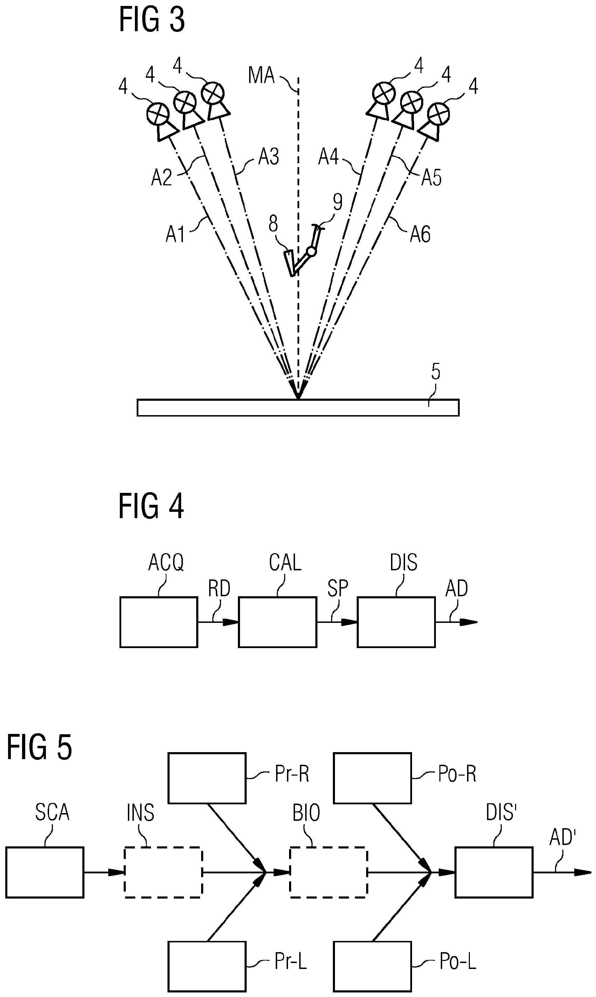

Die Röntgenstrahlungsquelle 4 ist dem Detektor 5 gegenüberliegend so angeordnet und ausgebildet, dass der Detektor 5 von ihr emittierte Röntgenstrahlung erfasst, nachdem zumindest ein Teil der Röntgenstrahlung die Brust O der Patientin durchdrungen hat. Es werden also Projektionen der Brust O als Rohdaten RD erfasst. Dabei ist die Röntgenstrahlungsquelle 4 relativ zum Detektor 5 mittels eines Dreharms 18 in einem Bereich von beispielsweise ± 25° um eine Grundstellung, d. h. um eine Mittelachse MA schwenkbar, in der sie senkrecht über der Detektionsfläche 5.1 steht (siehe auch FIG 3).

Zusätzlich weist das Tomosynthesegerät 2 eine Biopsieeinrichtung 8 als Untersuchungsinstrument 8 und einen Roboterarm 9 als Positionierungseinrichtung 9 auf. Der Roboterarm 9 ist an einem festen Ende mit der Quelle-Detektor-Anordnung 3 verbunden. Er weist Gelenke und motorische Elemente zur Positionierung der Biopsieeinrichtung 8 auf, die mit seinem freien Ende verbunden ist. Die Biopsieeinrichtung 8 umfasst einen Nadelhalter und eine darin gehaltene Biopsienadel (hier nicht dargestellt). Zur Visualisierung einer Biopsie, also einer Gewebeentnahme zur histologischen Untersuchung, mittels des Tomosynthesesystems 1 wird, wie weiter unten beschrieben, das erfindungsgemäße Verfahren zur Darstellung durchgeführt.

Das Rechnersystem 20 umfasst eine Rechnereinheit 12 und jeweils damit verbunden eine Maus 13, eine Tastatur 14 sowie einen Bildschirm 15. Der Bildschirm 15 dient hier als Anzeigeeinheit 15, Maus 13 und Tastatur 14 dienen jeweils als Eingabegerät. Die Rechnereinheit 12 umfasst eine Bildrekonstruktionseinrichtung 10 und eine Steuereinrichtung 11 (hier schematisch als Blöcke dargestellt) sowie ein Laufwerk 16 zum Einlesen von CD bzw. DVD. Dabei können die Bildrekonstruktionseinrichtung 10 und die Steuereinrichtung 11 gemeinsam Komponenten der Rechnereinheit 12 nutzen, wie z. B. Speicher, Prozessoren und dergleichen. Das Rechnersystem 20 kann in demselben Raum wie das Tomosynthesegerät 2 angeordnet sein, es kann sich aber auch in einem angrenzenden Kontrollraum oder in einer noch weiteren räumlichen Entfernung befinden.

FIG 2 zeigt ein Ausführungsbeispiel einer erfindungsgemäßen Bildrekonstruktionseinrichtung 10 schematisch als Blockdiagramm. Die Bildrekonstruktionseinrichtung 10 umfasst eine Eingangsdatenschnittstelle 24, eine Erfassungseinheit 21, eine Ermittlungseinheit 22, eine Ausgabeeinheit 23 sowie eine Ausgangsschnittstelle 25. Die einzelnen Komponenten der Bildrekonstruktionseinrichtung 10 sind miteinander über einen gemeinsamen Bus 26 verbunden, sodass Daten zwischen den einzelnen Komponenten je nach Bedarf frei ausgetauscht werden können. Die Eingangsdatenschnittstelle 24 kann beispielsweise über ein Netzwerk mit weiteren Speichereinrichtungen oder mit einem Tomosynthesegerät (siehe FIG 1) verbunden sein und kann davon übertragene Daten empfangen. Die Ausgangsschnittstelle 25 kann ebenfalls über ein Netzwerk mit weiteren Speichereinrichtungen verbunden sein oder aber die Ausgabedaten direkt auf einen Bildschirm, einen Beamer, einen Touchscreen oder dergleichen übertragen. Das Zusammenwirken der einzelnen Komponenten der Steuereinrichtung wird folgend anhand von FIG 4 und FIG 5 beschrieben.

In FIG 3 ist beispielhaft und schematisch eine erfindungsgemäße seitliche Anordnung der Akquisitionswinkel A1, A2, ..., A6 dargestellt. Zur Veranschaulichung ist die Röntgenquelle 4 schematisch in mehreren Positionen dargestellt, sodass ein von ihr emittierter mittlerer Röntgenstrahl je nach ihrer Position in einem definierten Akquisitionswinkel A1, A2, ..., A6 auf den Detektor 5 auftrifft. Senkrecht auf dem Detektor erstreckt sich eine Mittelachse MA. Im Bereich der Mittelachse MA sind die Biopsieeinrichtung 8 und deren Roboterarm 9 (hier klein angedeutet) angeordnet. Wäre die Röntgenquelle im Bereich der Mittelachse MA positioniert, würden die Biopsieeinrichtung 8 und der Roboterarm 9 den Strahlengang blockieren bzw. zu Artefakten bei der Bildgebung führen. Im Bereich der Mittelachse MA werden also keine Daten akquiriert. Erfindungsgemäß werden dementsprechend nur Rohdaten RD erfasst, die aus den seitlichen Akquisitionswinkeln A1, A2, ..., A6 akquiriert wurden. Die seitlichen Akquisitionswinkel A1, A2, ..., A6 sind schräg beidseits der Mittelachse MA und hier zugleich schräg zum Detektor 5 angeordnet. Sie nehmen dabei Winkel von A3, A4 = ±15°; A2, A5 = ±20° und A1, A6 = ± 25° zur Mittelachse MA ein. Bevorzugt werden je nach Bedarf zusätzlich Rohdaten RD in Zwischenpositionen (hier nicht dargestellt) akquiriert bzw. erfasst.

FIG 4 zeigt beispielhaft ein Blockdiagramm eines erfindungsgemäßen Verfahrens zur Darstellung. In einem ersten Schritt ACQ werden Rohdaten RD erfasst. Dazu werden die mit seitlichen Akquisitionswinkeln A1, A2, ..., A6 detektierten Projektionsaufnahmen über die Eingangsschnittstelle 24 von der Rekonstruktionseinheit 10 empfangen und über den Bus 26 an die Erfassungseinheit 21 weitergeleitet. Die Akquisition, welche nicht von dem erfindungsgemäßen Verfahren umfasst ist, kann dabei unmittelbar vor dem Verfahren erfolgen, sie kann jedoch auch zeitlich davon beabstandet ausgeführt werden.

Die Projektionsaufnahmen werden mit einer geringen Strahlendosis angefertigt, sodass für eine Beurteilung durch einen Betrachter aus den Rohdaten RD zunächst in einem zweiten Schritt CAL geeignete synthetische Projektionen SP ermittelt werden. Dies geschieht in der Ermittlungseinheit 22 vorzugsweise mittels einer direkten Rekonstruktion der synthetischen Projektionen SP aus den Rohdaten RD, deren Ergebnis je nach Bedarf eine Maximumintensitätsprojektion oder ein rotierendes Mammogramm sein kann. Es wird also explizit kein Zwischenschritt ausgeführt, in welchem hilfsweise ein Volumendatensatz rekonstruiert wird.

In einem dritten Schritt DIS werden die synthetischen Projektionen SP mittels der Ausgabeeinheit 23 über die Ausgangsschnittstelle 25 als Ausgabedaten AD ausgegeben, die das Untersuchungsobjekt O rotierend in einer Bildsequenz darstellen. Die einzelnen ermittelten synthetischen Projektionen SP werden also zu einer Bildsequenz AD zusammengeführt, die so geordnet ist, dass bei einem Durchlaufen bzw. Abspielen der Bildsequenz AD bei einem Betrachter der Eindruck entsteht, das dargestellte Untersuchungsobjekt O würde rotieren. Der Betrachter kann bei einer Ausgabe DIS auf einem Bildschirm 15 den Ablauf der Bildsequenz AD mit Hilfe eines Eingabemittels wie z. B. der Maus 13 oder der Tastatur 14 jederzeit beschleunigen, verlangsamen oder anhalten. Die Bildsequenz AD kann aber auch beispielsweise in einen Speicher der Rechnereinheit 12 oder über ein Netzwerk ausgegeben werden.

FIG 5 zeigt ein Blockdiagramm eines weiteren Ausführungsbeispiels eines erfindungsgemäßen Verfahrens zur Darstellung eines interessierenden Bereichs. Zunächst wird in einem Schritt SCA mittels eines bekannten Verfahrens ein vollständiger Tomosynthesescan erzeugt. Dieser wird zur Planung des folgenden Eingriffs, also bevorzugt zur Zielplanung und besonders bevorzugt auch zur Wegplanung für eine folgende Biopsie mit Hilfe einer Biopsieeinrichtung 8 mit einer Biopsienadel verwendet. Entsprechend dieser Planung wird in einem folgenden Schritt INS, der nicht vom erfindungsgemäßen Verfahren umfasst ist (gestrichelt dargestellt), die Biopsienadel in das Untersuchungsobjekt O eingebracht.

Um die Position der Biopsienadel visuell zu kontrollieren, wird folgend das bereits anhand von FIG 4 beschriebene Verfahren zweimal ausgeführt. Dazu wird in einem Schritt Pr-R der interessierende Bereich aus einem rechtsseitigen Winkelbereich und einem Schritt Pr-L aus einem linksseitigen Winkelbereich dargestellt. Die daraus erhaltenen Ausgabedaten AD (Pre-Fire-Daten) können einerseits, wie hier dargestellt, zu einer Gesamtausgabe am Ende des Verfahrens zwischengespeichert werden. Andererseits können sie zur unmittelbaren Kontrolle des erfolgreichen Einbringens INS der Biopsienadel zusätzlich auch gleich z. B. auf einem Bildschirm 15 ausgegeben werden. Dazu können die Pre-Fire-Daten auch in Kombination mit aus dem Tomosynthesescan erhaltenen Schichtbildern dargestellt werden, um dem Betrachter einen noch besseren räumlichen Eindruck zu vermitteln.

In einem folgenden Schritt BIO (gestrichelt dargestellt), der nicht von dem erfindungsgemäßen Verfahren umfasst ist, erfolgt die Probenentnahme BIO aus dem interessierenden Bereich mittels der Biopsienadel.

Anschließend wird wiederum das anhand von FIG 4 beschriebene erfindungsgemäße Verfahren zweimal ausgeführt. Dazu wird in einem Schritt Po-R der interessierende Bereich aus einem rechtsseitigen Winkelbereich und einem Schritt Po-L aus einem linksseitigen Winkelbereich dargestellt. Die daraus erhaltenen Ausgabedaten AD (Post-Fire-Daten) können, wie bereits anhand der Pre-Fire-Daten geschildert, ausgegeben werden. So kann einerseits der Erfolg der Probenentnahme BIO kontrolliert werden, andererseits können die Daten für die im Folgenden beschriebene Gesamtausgabe DIS' zwischengespeichert werden.

In einem weiteren Schritt DIS' erfolgt die Gesamtausgabe DIS' der Gesamtausgabedaten AD`. Die Gesamtausgabedaten AD' stellen dabei eine Dokumentation des gesamten Eingriffs dar. Sie umfassen dabei die Daten des Tomosynthesescans SCA, die als Schichtbildstapel hinterlegt sind, die rechtseitigen Pre-Fire-Daten, die linksseitigen Pre-Fire-Daten sowie die rechtseitigen Post-Fire-Daten und die linksseitigen Post-Fire-Daten. Durch die Zusammenstellung all dieser Daten kann dem Betrachter ein möglichst umfassender Eindruck des gesamten Eingriffs vermittelt werden.

Die unterschiedlichen Ansichten wirken dabei zusammen, indem sich die Vorteile bei der räumlichen Wahrnehmung der einzelnen Datensätze, also der Schichtdaten und der rotierend dargestellten Bildsequenzen, synergetisch ergänzen. Dadurch können der interessierende Bereich und ggf. auch die Position der Biopsienadel besser lokalisiert und beurteilt werden, als es bei einem einzelnen der Datensätze möglich wäre. Erfindungsgemäß werden dabei zugleich die von der Biopsienadel verursachten Artefakte bei der Bildgebung vermieden.

Es wird abschließend noch einmal darauf hingewiesen, dass es sich bei den vorhergehend detailliert beschriebenen Vorrichtungen und Verfahren lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können. Ebenso schließen die Begriffe "Einrichtung", "Einheit" und "System" nicht aus, dass die betreffende Komponente aus mehreren zusammenwirkenden Teilkomponenten besteht, die gegebenenfalls auch räumlich verteilt sein können.

### Bezugszeichenliste

1 Tomosynthesesystem
2 Tomosynthesegerät
3 Quelle-Detektor-Anordnung
4 Röntgenstrahlungsquelle
5 Detektor
5.1 Detektionsfläche
6 Platte
7 kreisförmige Ausnehmung
8 Untersuchungsinstrument, Biopsieeinrichtung
9 Roboterarm
10 Bildrekonstruktionseinrichtung
11 Steuereinrichtung
12 Rechnereinheit
13 Maus
14 Tastatur
15 Bildschirm
16 Laufwerk
17 Standsäule
18 Dreharms
20 Rechnersystem
21 Erfassungseinheit
22 Ermittlungseinheit
23 Ausgabeeinheit
24 Eingangsdatenschnittstelle
25 Ausgangsschnittstelle
26 Bus
A1, A2, ..., A6 Akquisitionswinkel
SP synthetische Projektionen
AD Ausgabedaten
AD`Gesamtausgabedaten
MA Mittelachse
O Untersuchungsobjekt, Brust
RD Rohdaten
ACQ, CAL, DIS Verfahrensschritte
BIO, DIS`, INS, Po-L, Po-R, Pr-L, Pr-R Verfahrensschritte

## Patentansprüche

1. Verfahren zur Darstellung eines interessierenden Bereichs innerhalb eines Untersuchungsobjekts (O), in das ein Untersuchungsinstrument (8) eingebracht ist, mittels eines Tomosynthesesystems (2),
wobei das Einbringen nicht vom erfindungsgemäßen Verfahren umfasst ist,
wobei das Verfahren zumindest folgende Schritte umfasst:
- Erfassen (ACQ) von Rohdatensätzen (RD) aus einer Anzahl von seitlichen Akquisitionswinkeln (A1, A2, A3, ..., A6), wobei Akquisitionswinkel in einem Bereich um eine Mittelachse ausgelassen werden und das Untersuchungsinstrument im Bereich der Mittelachse angeordnet ist,
wodurch ein Blockieren eines Strahlengangs durch das Untersuchungsinstrument vermieden wird,
wobei die Rohdatensätze (RD) mit Akquisitionswinkeln (A1, A2, A3, ..., A6) von mindestens 10°, bevorzugt mindestens 15°, zu einer Mittelachse (MA) erfasst werden,
- Ermitteln (CAL) einer Anzahl von synthetischen Projektionen (SP) aus den Rohdatensätzen (RD),
wobei die synthetischen Projektionen (SP) direkt aus den Rohdatensätzen (RD) rekonstruiert werden, oder
wobei das Ermitteln (CAL)ein Rekonstruieren eines Volumendatensatzes oder einzelner dünner Schichten aus den Rohdaten und ein Vorwärtsprojizieren des Volumendatensatzes oder der dünnen Schichten auf einen virtuellen Detektor umfasst, und
- Ausgabe (DIS) der synthetischen Projektionen (SP) als Bildsequenz (AD), welche das Untersuchungsobjekt (O) rotierend darstellt,
wobei die synthetischen Projektionen (SP) in der Bildsequenz (AD) derart zusammengeführt werden, dass bei einem Durchlaufen bzw. Abspielen der Bildsequenz (AD) bei einem Betrachter der Eindruck entsteht, das dargestellte Untersuchungsobjekt (O) würde rotieren.

2. Verfahren nach Anspruch 1, wobei die Rohdatensätze (RD) mit Akquisitionswinkeln (A1, A2, A3, ..., A6) von höchstens 60°, bevorzugt höchstens 45°, besonders bevorzugt höchstens 25°, zu einer Mittelachse (MA) erfasst werden.

3. Verfahren nach einem der vorstehenden Ansprüche, wobei die Anzahl der seitlichen Akquisitionswinkel (A1, A2, A3, ..., A6) mindestens 10, bevorzugt 20 beträgt.

4. Verfahren nach einem der vorstehenden Ansprüche, wobei zwei Rohdatensätze (RD) jeweils aus Akquisitionswinkeln (A1, A2, A3, ..., A6) erfasst werden, die einander bezogen auf eine Mittelachse gegenüberliegend angeordnet sind.

5. Verfahren nach einem der vorstehenden Ansprüche, wobei die Ausgabe (DIS) der synthetischen Projektionen (SP) als Maximumintensitätsprojektion umfasst.

6. Verfahren nach einem der vorstehenden Ansprüche, wobei die Rohdaten (RD) vor oder nach einer Betätigung des Untersuchungsinstruments (8) erfasst werden,
wobei es sich bei der Betätigung um den Vorgang einer Probenentnahme bei einer Biopsie mit Hilfe einer Biopsienadel handelt,
wobei die Probenentnahme nicht von dem erfindungsgemäßen Verfahren umfasst ist.

7. Bildrekonstruktionseinrichtung (10) zur Darstellung eines interessierenden Bereichs innerhalb eines Untersuchungsobjekts (O), in das ein Untersuchungsinstrument (8) eingebracht ist, umfassend
- eine Erfassungseinheit (21), die so ausgebildet ist, dass sie Rohdatensätze (RD) aus einer Anzahl von seitlichen Akquisitionswinkeln (A1, A2, A3, ..., A6) erfasst, wobei Akquisitionswinkel in einem Bereich um eine Mittelachse ausgelassen werden und das Untersuchungsinstrument im Bereich der Mittelachse angeordnet ist,
wobei die Rohdatensätze (RD) mit Akquisitionswinkeln (A1, A2, A3, ..., A6) von mindestens 10°, bevorzugt mindestens 15°, zu einer Mittelachse (MA) erfasst werden,
wodurch ein Blockieren eines Strahlengangs durch das Untersuchungsinstrument vermieden wird, - eine Ermittlungseinheit (22), die so ausgebildet ist, dass sie eine Anzahl von synthetischen Projektionen (SP) aus den Rohdatensätzen (RD) ermittelt,
wobei die synthetischen Projektionen (SP) direkt aus den Rohdatensätzen (RD) rekonstruiert werden, oder
wobei das Ermitteln (CAL)ein Rekonstruieren eines Volumendatensatzes oder einzelner dünner Schichten aus den Rohdaten und ein Vorwärtsprojizieren des Volumendatensatzes oder der dünnen Schichten auf einen virtuellen Detektor umfasst, und
- eine Ausgabeeinheit (23), die so ausgebildet ist, dass sie die synthetischen Projektionen (SP) als Bildsequenz (AD), welche das Untersuchungsobjekt (O) rotierend darstellt, ausgibt,
wobei die synthetischen Projektionen (SP) in der Bildsequenz (AD) derart zusammengeführt werden, dass bei einem Durchlaufen bzw. Abspielen der Bildsequenz (AD) bei einem Betrachter der Eindruck entsteht, das dargestellte Untersuchungsobjekt (O) würde rotieren.

8. Tomosynthesesystem (1) mit einem Tomosynthesegerät (2) mit einer Quelle-Detektor-Anordnung (3), die zur Aufnahme von Rohdaten (RD) eines Untersuchungsobjekts (O) aus definierten Akquisitionswinkeln (A1, A2, A3, ..., A6) ausgebildet ist, und einer Bildrekonstruktionseinrichtung (10) nach Anspruch 7.

9. Computerprogrammprodukt mit einem Computerprogramm, welches direkt in eine Speichereinrichtung einer Bildrekonstruktionseinrichtung (10) eines Tomosynthesesystems (1) ladbar ist, mit Programmabschnitten, um alle Schritte eines Verfahrens nach einem der Ansprüche 1 bis 6 auszuführen, wenn das Computerprogramm in der Bildrekonstruktionseinrichtung (10) des Tomosynthesesystems (1) ausgeführt wird.

10. Computerlesbares Medium, auf welchem von einer Rechnereinheit einlesbare und ausführbare Programmabschnitte gespeichert sind, um alle Schritte eines Verfahrens nach einem der Ansprüche 1 bis 6 auszuführen, wenn die Programmabschnitte von der Rechnereinheit ausgeführt werden.

## Claims

1. Method for visualising a region of interest inside an examination object (O) into which an examination instrument (8) has been introduced, the method being performed by means of a tomosynthesis system (2),
wherein the introduction is not included by the method according to the invention,
wherein the method comprises at least the following steps:
- acquiring (ACQ) raw datasets (RD) from a number of lateral acquisition angles (A1, A2, A3, ..., A6), wherein acquisition angles in a range around a central axis are omitted and the examination instrument is arranged in the vicinity of the central axis,
whereby a blocking of a beam path by the examination instrument is avoided,
wherein the raw datasets (RD) are acquired at acquisition angles (A1, A2, A3, ..., A6) of at least 10°, preferably at least 15°, to a central axis (MA),
- calculating (CAL) a number of synthetic projections (SP) from the raw datasets (RD),
wherein the synthetic projections (SP) are reconstructed directly from the raw data (RD), or
wherein the calculating (CAL) comprises a reconstruction of a volume dataset or individual thin slices from the raw data and a forward projection of the volume dataset or the thin slices on a virtual detector, and
- outputting (DIS) the synthetic projections (SP) in the form of an image sequence (AD) which represents the examination object (O) in a rotating visualisation,
wherein the synthetic projections (SP) are merged in the image sequence (AD) in such a manner that, when the image sequence (AD) is run or played back, the impression is created in an observer that the visualised examination object (O) is rotating.

2. Method according to claim 1, wherein the raw datasets (RD) are acquired at acquisition angles (A1, A2, A3, ..., A6) of at most 60°, preferably at most 45°, particularly preferably at most 25°, to a central axis (MA).

3. Method according to one of the preceding claims, wherein the number of lateral acquisition angles (A1, A2, A3, ..., A6) amounts to at least 10, preferably 20.

4. Method according to one of the preceding claims, wherein two raw datasets (RD) are acquired in each case from acquisition angles (A1, A2, A3, ..., A6) which, referred to a central axis, are arranged opposite each other.

5. Method according to one of the preceding claims, wherein the output (DIS) of the synthetic projections (SP) comprises a maximum intensity projection.

6. Method according to one of the preceding claims, wherein the raw data (RD) is acquired before and/or after an actuation of the examination instrument (8),
wherein the actuation entails the sample-taking process during a biopsy with the aid of a biopsy needle,
wherein the sample-taking is not included by the method according to the invention.

7. Image reconstruction device (10) for visualising a region of interest inside an examination object (O) into which an examination instrument (8) has been introduced, comprising
- an acquisition unit (21) which is embodied in such a way that it acquires raw datasets (RD) from a number of lateral acquisition angles (A1, A2, A3, ..., A6), wherein acquisition angles in a range around a central axis are omitted and the examination instrument is arranged in the vicinity of the central axis,
wherein the raw datasets (RD) are acquired at acquisition angles (A1, A2, A3, ..., A6) of at least 10°, preferably at least 15°, to a central axis (MA),
whereby a blocking of a beam path by the examination instrument is avoided,
- a calculation unit (22) which is embodied in such a way that it calculates a number of synthetic projections (SP) from the raw datasets (RD),
wherein the synthetic projections (SP) are reconstructed directly from the raw data (RD), or
wherein the calculating (CAL) comprises a reconstruction of a volume dataset or individual thin slices from the raw data and a forward projection of the volume dataset or the thin slices on a virtual detector, and
- an output unit (23) which is embodied in such a way that it outputs the synthetic projections (SP) in the form of an image sequence (AD) which represents the examination object (O) in a rotating visualisation,
wherein the synthetic projections (SP) are merged in the image sequence (AD) in such a manner that, when the image sequence (AD) is run or played back, the impression is created in an observer that the visualised examination object (O) is rotating.

8. Tomosynthesis system (1) comprising a tomosynthesis device (2) having a source-detector arrangement (3) which is embodied for acquiring raw data (RD) of an examination object (O) from defined acquisition angles (A1, A2, A3, ..., A6), and an image reconstruction device (10) according to claim 7.

9. Computer program product having a computer program which can be loaded directly into a memory device of an image reconstruction device (10) of a tomosynthesis system (1), and having program sections for the purpose of performing all steps of a method according to one of claims 1 to 6 when the computer program is executed in the image reconstruction device (10) of the tomosynthesis system (1).

10. Computer-readable medium on which are stored program sections that can be read in and executed by a computer unit in order to perform all steps of a method according to one of claims 1 to 6 when the program sections are executed by the computer unit.

## Revendications

1. Procédé de représentation, au moyen d'un système (2) de tomosynthèse de représentation d'une partie à laquelle on s'intéresse au sein d'un objet (O) en examen, dans lequel un instrument (8) d'examen est introduit,
dans lequel l'introduction ne fait pas partie du procédé suivant l'invention,
dans lequel le procédé comprend au moins les stades suivants :
- saisie (ACQ) d'ensembles (RD) de données brutes, à partir d'un nombre d'angles (A1, A2, A3, ..., A6) d'acquisition latéraux, dans lequel on donne libre cours à des angles d'acquisition dans une partie autour d'un axe médian et l'instrument d'examen est disposé dans la partie de l'axe médian,
grâce à quoi on empêche un blocage d'un chemin de rayonnement dans l'instrument d'examen,
dans lequel on saisit les ensembles (RD) de données brutes avec des angles (A1, A2, A3, ..., A6) d'acquisition d'au moins 10°, de préférence d'au moins 15°, par rapport à un axe (MA) médian,
- détermination (CAL) d'un nombre de projections (SP) synthétiques à partir des ensembles (RD) de données brutes,
dans lequel on reconstruit des projections (SP) synthétiques directement à partir des ensembles (RD) de données brutes, ou dans lequel la détermination (CAL) comprend une reconstruction d'un ensemble de données en volume ou de tranches minces individuelles à partir des données brutes et une projection vers l'avant de l'ensemble de données en volume ou des tranches minces sur un détecteur virtuel, et
- sortie (DIS) des projections (SP) synthétiques sous la forme d'une séquence (AD) d'images, qui représente tournant l'objet (O) en examen,
dans lequel on réunit les projections (SP) synthétiques en la séquence (AD) d'image, de manière à créer, lors d'un passage ou d'un déroulement de la séquence (AD) d'images, pour un observateur, l'impression que l'objet (O) en examen représenté serait tournant.

2. Procédé suivant la revendication 1, dans lequel on saisit les ensembles (RD) de données brutes avec des angles (A1, A2, A3, ..., A6) d'acquisition de 60° au plus, de préférence de 45° au plus, d'une manière particulièrement préférée de 25° au plus, par rapport à un axe (MA) médian.

3. Procédé suivant l'une des revendications précédentes, dans lequel le nombre des angles (A1, A2, A3, ..., A6) de d'acquisition latéraux est d'au moins 10, de préférence 20.

4. Procédé suivant l'une des revendications précédentes, dans lequel on saisit deux ensembles (RD) de données brutes, chacun à partir d'angles (A1, A2, A3, ..., A6) d'acquisition, qui sont disposés l'un par rapport à l'autre en opposition rapporté à un axe médian.

5. Procédé suivant l'une des revendications précédentes, dans lequel la sortie (DIS) des projections (SP) synthétiques s'effectue sous la forme d'une projection d'intensité maximum.

6. Procédé suivant l'une des revendications précédentes, dans lequel on saisit les données (RD) brutes avant ou après un actionnement de l'instrument (8) d'examen,
dans lequel l'actionnement est une opération de prélèvement d'échantillon, lors d'une biopsie à l'aide d'une aiguille de biopsie,
dans lequel le prélèvement d'échantillon ne fait pas partie du procédé suivant l'invention.

7. Dispositif (10) de reconstruction d'image, pour la représentation d'une partie à laquelle on s'intéresse au sein d'un objet (O) en examen, dans lequel est introduit un instrument (8) d'examen, comprenant
- une unité (21) de saisie, qui est constituée de manière à saisir des ensembles (RD) de données brutes à partir d'un nombre d'angles (A1, A2, A3, ..., A6) d'acquisition latéraux, dans lequel on donne libre cours aux angles d'acquisition dans une partie autour d'un axe médian et l'instrument d'examen est disposé dans la partie de l'axe médian,
dans lequel on saisit les ensembles (RD) de données brutes avec des angles (A1, A2, A3, ..., A6) d'acquisition d'au moins 10°, de préférence d'au moins 15° par rapport à un axe (MA) médian, grâce à quoi on empêche un blocage d'un chemin de rayonnement dans l'instrument d'examen,
- une unité (22) de détermination, qui est constituée de manière à déterminer un nombre de projections (SP) synthétiques à partir des ensembles (RD) de données brutes,
dans lequel on reconstruit les projections (SP) synthétiques directement à partir des ensembles (RD) de données brutes, ou dans lequel la détermination (CAL) comprend un reconstruction d'un ensemble de données en volume ou de tranches minces individuelles à partir des données brutes et une projection vers l'avant de l'ensemble de données en volume ou des tranches minces sur un détecteur virtuel, et
- une unité (23) de sortie, qui est constituée de manière à sortir les projections (SP) synthétiques sous la forme d'une séquence (AD) d'images, qui représentent tournant l'objet (O) en examen,
dans lequel on rassemble les projections (SP) synthétiques en la séquence (AD) d'images, de manière à créer, lors d'un passage ou
d'un déroulement de la séquence (AD) d'images, pour un observateur, l'impression que l'objet (O) en examen représenté serait tournant.

8. Système (1) de tomosynthèse comprenant un appareil (2) de tomosynthèse ayant un agencement (3) source-détecteur, qui est constitué pour l'enregistrement de données (RD) brutes d'un objet (O) à examiner à partir d'angles (A1, A2, A3, ..., A6) d'acquisition définis, et un dispositif (10) de reconstruction d'image suivant la revendication 7.

9. Produit de programme d'ordinateur comprenant un programme d'ordinateur, qui peut être chargé directement dans un dispositif de mémoire d'un dispositif (10) de reconstruction d'image d'un système (1) de tomosynthèse, pour exécuter tous les stades d'un procédé suivant l'une des revendications 1 à 6, lorsque le programme d'ordinateur est exécuté dans le dispositif (10) de reconstruction d'image du système (1) de tomosynthèse.

10. Support, déchiffrable par ordinateur, sur lequel sont mises en mémoire des parties de programme, déchiffrables et exécutables par une unité informatique, pour exécuter tous les stades d'un procédé suivant l'une des revendications 1 à 6, lorsque les parties de programme sont exécutées par l'unité informatique.
